# EUROPEAN PATENT APPLICATION

(11) **EP 0 909 811 A2**
(43) Date of publication of application: **21.04.1999**
(21) Application number: 98119487.1
(22) Date of filing: 15.10.1998
(51) Int. Cl.: C12M 3/06

(54) **Bioreactor for culturing animal and/or human cells, particularly hepatocytes**

(30) Priority: 16.10.1997 IT MO970182
(71) Applicant: B. BRAUN CAREX S.p.A., 41037 Mirandola (Modena) (IT)
(72) Inventor: Gavioli, Giuliana, 41037 Mirandola (Modena) (IT)
(74) Representative: Modiano, Guido, Dr.-Ing.

(57) **Abstract**

The bioreactor for culturing animal and/or human cells, particularly hepatocytes, comprises a container provided with at least one inlet and at least one outlet for blood or plasma, inside which at least one macroporous cell supporting body is accommodated; the body is internally hollow and has internal and external surfaces covered by filtering means, and ducts are provided for conveying the cells from outside to the macroporous supporting body.

## Description

The present invention relates to a bioreactor for culturing animal and/or human cells, particularly hepatocytes.

The use of bioartificial organs for clinical applications in man is known.

In recent years, models of bioreactors have been developed and used both experimentally and clinically which are based on the ability to accommodate mammalian cells which express differentiated functions.

One of the bioartificial organs most involved in experiments is the bioartificial liver, which with different bioengineering solutions has the fundamental goal of obtaining a cell culture of hepatocytes exhibiting effective and constant metabolism and protein synthesis.

This goal must be met by providing a physical separation between the cell culture and the plasma or blood of the patient to be treated, such as to allow solute and gas exchanges on the one hand and prevent, on the other hand, the transfer of the cells to the patient, in order to avoid immunization phenomena in said patient.

Achieving this goal entails several problems, at least three of which are highly important.

A first problem is the ability to provide a cell mass which is such as to be able to replace the hepatic function.

The cell mass is strictly dependent on the useful space reserved for the cell culture in the bioreactor and on the manner in which the cells are supported.

If it is necessary to provide a supporting carrier, said carrier usually occupies approximately two thirds of the volume available for the cell mass.

A second problem is the need to provide effective exchange between the cell mass and the patient.

This goal is highly dependent on the means used, which is usually a membrane, as a surface for exchange between the plasma and the cells, and on the consequent flow that is necessary in order to allow exchanges between the plasma or blood and the cells to occur.

A third problem is the speed with which the bioreactor can be prepared for clinical use on the patient in a very short time, in order to be able to act as quickly as possible in the acute stages of the patient's disorders.

According to the state of the art, up to now, in the clinical field the first bioreactors used have employed diffusion through dialysis membranes as a solute exchange method, initially using as bioreactors actual flat-fiber dialyzers (K.N. Matsamura et al., Alin Foundation, 1987) and then using filters containing hollow fibers (R. Williams, N.L. Sussman et al, Hepatrix Inc., Hepatology, 1996; A. Demetriou et al, Grace, Hepatology, 1993; A. Demetriou et al., Grace, Annals of Surgery, 1997) with or without oxygenation of the plasma meant for the hepatocyte culture.

The principle of perfusion in bioreactors based on hollow fibers has been used more recently (J. Gerlak, B. Braun Melsungen AG, Transplantation, 1994), achieving a more uniform distribution and a more complete exchange of solutes together with direct oxygenation of the hepatocytes inside the bioreactor.

Many studies of experimental bioreactors used in vitro have been published recently and are based on concepts which are different from the preceding ones, such as for example the artificial liver constituted by monolayers of polar hepatocytes (R. Tompkins, M. Yarmush, Transplantation Review, 1993; A. Bader, Artificial Organs, 1995) or cultures of hepatocytes accommodated in the hollow fibers (F. Cerra, Journal of Surgical Results, 1992), or receptacles for cell cultures constituted by porous resins (N. Ohshimura, Kaneka Corp., Biotechnology and Bioengineering, 1994) or non-woven fabrics (M. Chableau, Journal of Hepatology, 1997).

All bioreactors based on hollow fibers currently used in the treatment of acute fulminating hepatitis as substrates for cell cultures have a limited capacity in terms of exchange (so-called "mass transfer").

The plasma or blood of the patient reaches the extrafiber space, which is filled with hepatocytes, by passing through said fibers only when applying a high pressure, which damages the cell culture.

Moreover, due to the high axial resistance of the flow, exchange capacity decreases in proportion in the bioreactor and is reduced considerably in the distal part of said bioreactor; this causes the distal portion of the bioreactor to be practically unused.

It should be added to the above that hollow-fiber bioreactors can accommodate a relatively small amount of cells, while it is acknowledged that at least 200-300 grams of hepatocytes are necessary in order to compensate the compromised hepatic function.

The aim of the present invention is to solve the above problems of the prior art, by providing a bioreactor for culturing animal and/or human cells, particularly hepatocytes, which allows to produce the necessary amount of said cells in order to replace the hepatic function, does not require intense pressures to activate its operation, and can be used completely throughout its structure.

This aim and other objects which will become apparent hereinafter are achieved by a bioreactor for culturing animal and/or human cells, particularly hepatocytes, characterized in that it comprises a container provided with at least one inlet and at least one outlet for blood or plasma, inside which at least one macroporous cell supporting body is accommodated, said body being internally hollow and having internal and external surfaces covered by filtering means, ducts being provided for conveying the cells from outside to said macroporous supporting body.

Further characteristics and advantages of the invention will become apparent from the following detailed description of a preferred embodiment of a bioreactor for culturing animal and/or human cells, particularly hepatocytes, illustrated only by way of non-limitative example in the accompanying drawings, wherein:

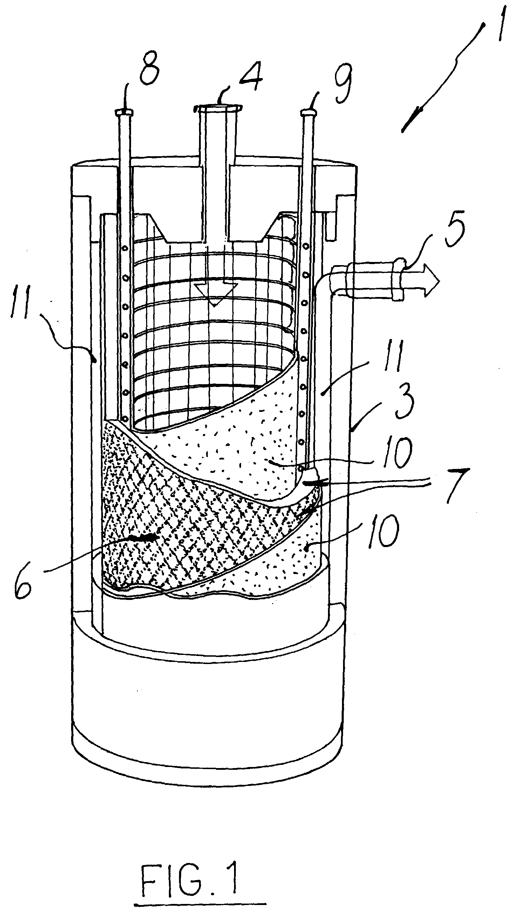

Figure 1 is a partially cutout view of a bioreactor for culturing animal and/or human cells, particularly hepatocytes, according to the invention;

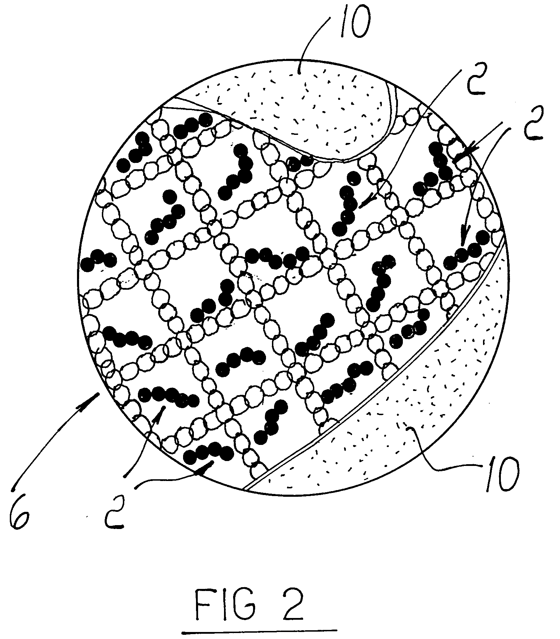

Figure 2 is an enlarged-scale schematic view of a detail of the structure that composes said bioreactor.

With particular reference to the above figures, the reference numeral 1 generally designates a bioreactor for culturing animal and/or human cells, particularly hepatocytes, schematically designated by the reference numeral 2, which comprises a container 3 provided with at least one inlet 4 arranged in the top region and with at least one outlet 5 for the blood or plasma of a patient to be treated.

Inside the container 3 there is provided at least one body 6 having a macroporous structure which is suitable to act as a support for the cells 2, is internally hollow and has internal and external surfaces covered by filtering means 7; both the container 3 and the body 6 preferably have a cylindrical shape and are internally hollow and arranged coaxially.

The bioreactor 1 is further provided with ducts, two in the specific case, designated by the reference numerals 8 and 9, for conveying the cells 2 from outside to said macroporous supporting body 6.

The filtering means 7 are constituted by two semipermeable membranes 10 which have a porosity between 0.1 and 0.5 microns when using plasma and between 0.1 and 10 microns when using blood.

For best operation of the bioreactor 1, the outlet 5 is arranged on one side of the container 3, while the inlet is centered coaxially with respect to said body 6.

By virtue of an intentional difference between the diameters of the macroporous supporting body 6 and of the container 3, a perimetric chamber 11 is formed between the internal surface of said container and said body 6 and is connected to the outlet 5.

The operation of the invention is as follows: the blood or plasma to be treated, which arrives from the patient, is introduced under slight pressure in the bioreactor through the inlet 4 and more specifically reaches the inside of the body 6, which has a macroporous structure.

The hepatocytes 2, introduced beforehand through the ducts 8 and 9, are supported by adhesion on said body.

Said blood (or plasma) first crosses, with a radially orientated stream, the innermost semipermeable membrane 10; then it passes through the macroporous body 6 and makes direct contact with the cells (hepatocytes) that are present on said body, restoring the correct metabolic values, and finally crosses the second external membrane 10, which is suitable to retain any cells that follow the stream. Said stream is purified at this point, is collected in the perimetric chamber 11 and leaves the bioreactor 1 through the outlet 5 to be returned to the patient.

During in vitro experiments, the hepatocytes 2 were inoculated into the macroporous body 6 of the bioreactor 1.

Said hepatocytes were perfused at a rate of 20 ml/min with serum-free Dulbecco Medium at a temperature of 37°C, oxygenated at 20%, supplemented with 100 µg/l of insulin, 34.83 µg/l of glucagon, 67 µg/l of dexamethasone and oligoelements, to which a solution of ammonium chloride at a concentration of 1 mM was added.

Ammonium removal was measured with the indophenol method and urea synthesis was measured with the diacetyl monoxime method after 12 hours of experimental study.

At the end of the experiment, the bioreactor 1 was opened and the hepatocytes 2 attached to the macroporous body 6 were examined by transmission electron microscopy (TEM) and by scanning electron microscopy (SEM).

The result was an increase in the production of urea per mg/1 x 10⁶ cells as follows:
0.133 mg (84%) at 4 hours;
0.143 mg (98%) at 8 hours;
0.152 mg (109%) at 12 hours.

Ammonium was removed with a constant stream per mg/l x 10⁶ cells as follows:
0.045 mg (4%) at 4 hours;
0.049 mg (12%) at 8 hours;
0.052 mg (19%) at 12 hours.

The use of TEM and SEM methods allowed to verify that most of the hepatocytes were attached to the macroporous body 6, exhibiting the typical characteristics of functioning hepatocytes, such as the presence of the endoplasmic reticulum, glycogen granules, well-differentiated mitochondria and formation of biliary canaliculi between adjacent hepatic cells.

It has thus been observed that the described invention achieves the intended aim.

The invention thus conceived is susceptible of modifications and variations, all of which are within the scope of the inventive concept.

All the details may further be replaced with other technically equivalent elements.

In the practical execution of the invention, the materials employed, as well as the shapes and the dimensions, may be any according to requirements without thereby abandoning the scope of protection of the appended claims.

The disclosures in Italian Patent Application No. MO97A000182 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A bioreactor for culturing animal and/or human cells, particularly hepatocytes, characterized in that it comprises a container which is provided with at least one inlet and at least one outlet for blood or plasma, inside which at least one macroporous cell supporting body is accommodated, said body being internally hollow and having internal and external surfaces covered by filtering means, ducts being provided for conveying the cells from outside to said macroporous supporting body.

2. The bioreactor according to claim 1, characterized in that said filtering means are constituted by semipermeable membranes.

3. The bioreactor according to claims 1 and 2, characterized in that said semipermeable membranes have a porosity between 0.1 and 0.5 microns for use with plasma and between 0.1 and 10 microns for use with blood.

4. The bioreactor according to claim 1, characterized in that said outlet is arranged on a side of the container.

5. The bioreactor according to the preceding claims, characterized in that between said macroporous supporting body and the internal surface of said container there is provided a perimetric chamber which is connected to said outlet.

6. The bioreactor according to the preceding claims, characterized in that said inlet leads into said macroporous supporting body.

7. The bioreactor according to the preceding claims, characterized in that the stream of blood or plasma to be treated is radial and passes substantially at right angles through said membranes and said macroporous body.

8. The bioreactor according to the preceding claims, characterized in that said macroporous body is constituted by biocompatible polymeric material.

9. The bioreactor according to claim 8, characterized in that said biocompatible material has a stratified lattice-like woven structure with connecting filaments between the layers of the nonwoven type.

10. The bioreactor according to claims 8 and 9, characterized in that said polymeric material is selected from the group consisting of polyester, polysulfone and polyolefins.

11. The bioreactor according to the preceding claims, characterized in that said macroporous body has a porosity which is equal to, or greater than, 250 microns.

12. The bioreactor according to the preceding claims, characterized in that said cells adhere directly to said macroporous body.

13. The bioreactor according to the preceding claims, characterized in that the volume of said macroporous body is between 8 and 15% of the volume of the cell mass that can be cultured.

14. The bioreactor according to the preceding claims, characterized in that said container and said macroporous body have a cylindrical shape and are mutually coaxial.
